Europäisches Patentamt

(19) European Patent Office

Office européen des brevets

(11) Publication number : **0 428 519 B1**

# (12) EUROPEAN PATENT SPECIFICATION

(45) Date of publication of patent specification :
16.06.93 Bulletin 93/24

(51) Int. Cl.⁵ : **C12N 5/10,** C12N 15/00, C12N 7/00

(21) Application number : **89905491.0**

(22) Date of filing : **12.04.89**

(86) International application number :
**PCT/US89/01526**

(87) International publication number :
**WO 89/09816 19.10.89 Gazette 89/25**

(54) METHOD FOR MANIPULATION OF THE CELL TYPES OF EUKARYOTES.

(30) Priority : **12.04.88 US 180548**
**02.06.88 US 201762**

(43) Date of publication of application :
**29.05.91 Bulletin 91/22**

(45) Publication of the grant of the patent :
**16.06.93 Bulletin 93/24**

(84) Designated Contracting States :
**AT BE CH DE FR GB IT LI LU NL SE**

(56) References cited :
**Virology, vol. 127, no. 1, 1983, Academic Press, Inc., C.A. Petit et al.: "Immortalization of rodent embryo fibroblasts by SV40 is maintained by the A gene", pages 74-82**
**Biological Abstracts/RRM, vol. 36, G. Almazan et al.: "Immortalization of an oligodendrocyte precursor cell via retrovirus carrying a temperature sensitive ocogene", abstract no. 28937, & 18th Annual Meeting of the Society for Neuroscience, Toronto, Ontario, CA, 13-18 November 1988, Soc. Neurosci Abstr 14(2). 1988. 1130**

(56) References cited :
**Molecular and Cellular Biology, vol. 6, no. 4, April 1986, American Society for Microbiology, P.S. Jat et al.: "Recombinant retroviruses encoding Simian Virus 40 large T antigen and polyomavirus large and middle T antigens", pages 1204-1217**
**Cell, vol. 28, 1982, pages 907-919, H. Beug et al.**

(73) Proprietor : **MASSACHUSETTS INSTITUTE OF TECHNOLOGY**
**77 Massachusetts Avenue**
**Cambridge, MA 02139 (US)**

(72) Inventor : **McKAY, Ronald, D., G.**
**3 Gorham Avenue**
**Brookline, MA 02146 (US)**
Inventor : **JAT, Parmjit, S.**
**Flat G 12-15 Rhiedol Terrace Islington**
**London N1 (GB)**
Inventor : **LAMAZAN, Guillermina**
**351 Harvard Street**
**Cambridge, MA 02138 (US)**

(74) Representative : **Holdcroft, James Gerald, Dr. et al**
**Graham Watt & Co., Riverhead**
**Sevenoaks, Kent TN13 2BN (GB)**

## Description

### Background

The mechanisms which generate differences between cell types are poorly understood. Every tissue in a eukaryote is made up of a mixture of cell types. The intrinsic molecular properties of these cell types and the interaction between cells give rise to the specialized functions played by each tissue in an organism. A wide range of newly developed methods are now employed to study the biochemistry of cellular differentiation in eukaryotes.

Many cell types can be taken from animals and grown in tissue culture for a short period of time. However, these primary cells have a limited lifespan in culture. The methods currently available for growing animal cells indefinitely in culture generally rely on tumor-derived cells, or on transformation of primary cells involving heritable changes which alter the growth state of the cells. The usefulness of permanently transformed cells may be limited because of characteristics introduced by the transformation process. It would be useful to have a means of producing or growing quantities of normal cells with defined characteristics.

### Summary of the Invention

The present invention relates to a method of producing conditionally immortalized continuous cell lines from preselected progenitor or precursor cells from vertebrates. The invention also relates to a method of deriving differentiated cells from the conditionally immortalized cell lines. In addition, the invention relates to recombinant retroviruses useful in the present invention.

According to the method of the present invention, a gene which confers the ability to grow continuously in culture is introduced, using known techniques, into a vertebrate cell. The function of the growth promoting, or immortalizing gene is controlled by an external factor or factors so that the gene's function or action may be regulated at will. When the immortalizing gene is altered in its function or action, the cell in which it resides switches to a differentiated state. According to the method of the present invention, large quantities of selected precursor cells can be grown and then induced to differentiate into a pre-selected derivative cell type.

In one embodiment of this invention, the growth promoting gene is a temperature sensitive (ts) mutant of the T antigen from the simian virus SV40 (tsA58). In this embodiment, the temperature sensitive mutant T antigen is introduced into normal neuroepithelial precursor cells, which are then maintained under conditions permissive for continuous growth and expansion of the cell population. Permissive conditions include a temperature at which the mutant SV40 T antigen functions to immortalize cell lines (in this case 33°C). When the cell population has reached the desired size, the temperature is shifted to one at which the growth promoting activity of the mutant SV40 gene is inactivated (39°C). In the absence of the growth promoting activity of the immortalizing gene the cells are induced to differentiate.

This invention therefor provides a general method for generating from selected precursor cells continuous cell lines which are capable of proliferation and subsequent differentiation.

### Brief Description of the Drawing

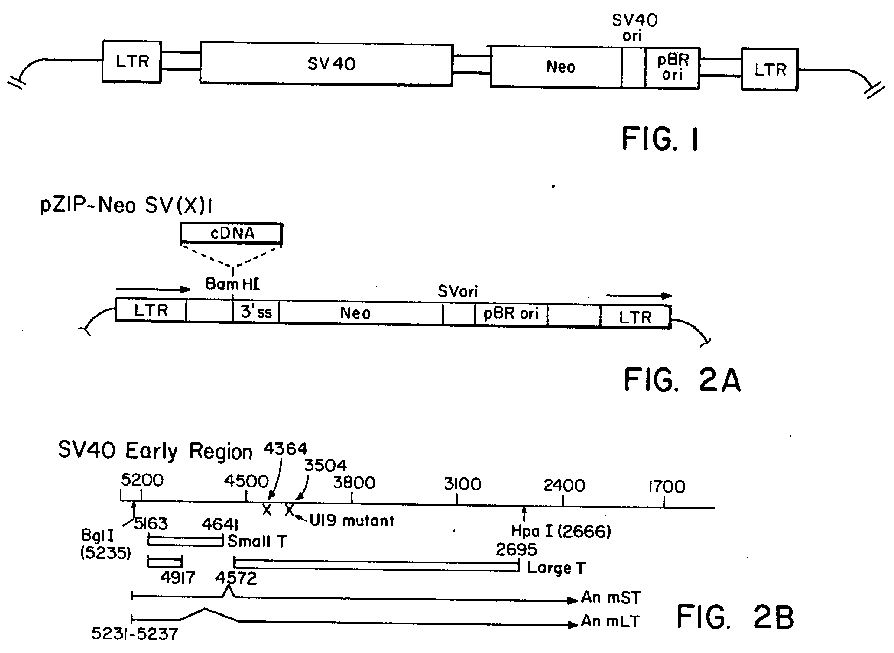

Figure 1 is a schematic representation of the plasmid pZipSV40tsA58, in which the genomic BglI-Hpa I fragment (nucleotides 5235-2666) derived from the early region of the SV40 mutant tsA58 was inserted into the shuttle vector pZipNeoSV(X)1 (Cepko, C. et al., Cell, 1984) with Bam HI linkers.

Figure 2 is a schematic representation of the construction of plasmid pZiptsa58U19. Figure 2A is a schematic representation of plasmid pZipNeoSV(X)1 (Cepko, C. et al., Cell, 1984). Figure 2B is a schematic representation of the hybrid of the SV40 T antigen gene, U19, which was constructed using the pZiptsA58 and the U19 mutant.

### Detailed Description of the Invention

The present invention relates to a novel method of immortalizing cell lines of selected progenitor cells, as well as to cell lines immortalized by the method and to recombinant retroviruses which include a growth promoting gene under external control, such as the temperature sensitive SV40 oncogene. In general, the method comprises introducing into cells of a selected type, in primary culture, a growth promoting gene, which enables the cells to grow continuously, and whose function is controlled by an external factor. The primary cells have been previously identified as representing proliferating precursor or progenitor cells. A second gene encoding a selectable marker (e.g., drug resistance) is also introduced into the cells, thereby making it possible to select cells which contain the growth promoting gene. Cells containing both the selectable marker and the growth promoting gene are selected (e.g., by plating onto media containing the drug against which the gene confers resistance) and isolated. It is to be understood that it is unnecessary to introduce a gene encoding a selectable marker if there are other means available for identifying and isolating the cells containing the growth promoting gene. The isolated cells are subsequently plated onto an appropriate medium, under conditions (e.g., temperature) under which the growth promoting function of the gene is activated (e.g., permissive conditions). After allowing sufficient time for the cells to proliferate, the conditions (e.g., temperature) are switched to nonpermissive conditions (e.g., increased temperature) to inactivate the growth promoting gene. Inactivation of the growth promoting gene allows the cells to differentiate.

In a particular embodiment of the present invention, the vertebrate cells are neuronal precursor cells and the temperature sensitive T antigen of SV40 tsA58 is the growth promoting gene. The temperature sensitive domain of the growth promoting gene was inserted into a recombinant retroviral vector by homologous recombination with a wild-type SV40 T antigen resident in the vector. Infection with the retroviral vector harboring the mutant T antigen made it possible to introduce the temperature sensitive growth promoting gene with high efficiency into primary cells. Tegtmeyer, P. et al., Journal of Virology, 16:168-178 (1975).

Primary cells used in the work described below and in the Examples were cells from the nervous system of a mammal. However, it is to be understood that the method of the present invention can be used to immortalize any type of vertebrate cell of interest. In addition, although the description of the method of the present invention makes specific reference to the use of a retroviral vector for introduction of a growth promoting gene which is an oncogene, it is to be understood that other known methods of gene transfer (e.g., electroporation, microinjection) can also be used for this purpose. It is also to be understood that the growth promoting gene can be any gene which effects immortalization or continuous growth of the cell type of interest. It is also possible to introduce conditionally immortalized cells into animals (e.g., mammals) and thereby produce transgenic animals in which the growth promoting gene is inactive at normal body temperatures. Because the ts or otherwise conditionally immortalized cells are present in such animals at the nonpermissive temperature, the precursor cells should be able to differentiate in vivo.

The method of the present invention has been carried out in cells from the developing vertebrate nervous system. Monoclonal antibodies have been generated which recognize the major cell types in the embryonic rat central nervous system (CNS). S. Hockfield and R.D.G. McKay, J. Neurosci, 5:3310-3328 (1985). Quantitative methods to determine the number of cells in the rat CNS on each day of embryonic development have also been developed. Knowledge of the number and types of cells, combined with methods which identify proliferating cells, allows identification of a neuroepithelial cell population with properties expected of a progenitor or precursor cell. K. Frederiksen and R.D.G. McKay, J. Neurosci, 8:1144-1151 (1988).

Because the cell types present in the developing brain had previously been characterized, it was possible to verify that differentiated cells produced by the method do, in fact, have the characteristics of neurons or glia. K. Frederiksen and R.D.G. McKay, J. Neurosci, 8:1144-1151 (1988); S. Hockfield and R.D.G. McKay, J. Neurosci, 5:3310-3328 (1985). This ability to characterize the cell types allows direct verification of the ability of the present method to produce a continuous cell line from which pre-selected differentiated derivatives can be produced through control of the growth promoting gene.

In the examples, two regions of the embryonic rat nervous system were chosen as sources of primary cells: the hippocampus and the cerebellum. Primary cells were plated in tissue culture and exposed to a synthetic retrovirus (see Example 1) carrying the temperature sensitive form of SV40 T antigen. The retrovirus also contains a gene encoding drug resistance (i.e., G418 resistance), which served as the basis for selecting cells containing the retrovirus. Immortal cell lines expressing SV40 T antigen were obtained from these cultures by growing the cells at 33°C in drug-containing medium. Those cells containing the G418 resistance gene (Neo) grew in the drug-containing medium; those lacking the gene did not survive. At 33°C, the surviving cells grew with the standard morphology of continuous cell lines. Clonal cells were obtained by picking colonies using cloning rings. The cells were then tested for their response to growth at 39°C, the nonpermissive temperature for tsA58 T antigen. The criteria evaluated for cellular response were morphology, immunohistochemistry and immunochemistry. The method used and the data obtained are described in detail in the Examples and demonstrate that when the temperature is raised, cells can differentiate to a cell type characteristic of neurons and glia. The methods used in verifying that the resulting differentiated cells have such characteristics are also described in the Examples.

A continuous precursor cell line which differentiates into mature oligodendrocytes has also been produced, using an approach similar to that described above. Oligodendrocytes, the myelin-forming cells of the CNS, develop during the first week of postnatal life in the optic nerve of the rat. Oligodendrocyte progenitors are bipotential cells which also give rise to type 2 astroctyes. To obtain permanent cell lines, primary cultures from the optic nerve of 3-day-old Sprague Dawley (SD) rats were infected with a retrovirus vector transducing the SV40 tsA58/U19 large T antigen (E. Paucha, et al., J. Virol., 57: 50-64 (1986). At the permissive temperature, one of the derived cell lines proliferates and expresses the T antigen and small amounts of GC (galactocerebroside), a surface lipid marker for oligodendrocytes and $A_2B_5$ (surface gangliosides). At the non-permissive temperature, the cells lose the T antigen expression, stop proliferating, and stain very strongly with GC and $A_2B_5$ antibodies. The two main protein components of myelin, MBP (myelin basic protein) and PLP (proteolipid protein) are also expressed. $A_2B_5$ and GC are selectable markers which are highly specific for either precursor or mature oligodendrocytes (e.g., GC is a lipid present only on oligodendrocytes). MB and PLP are two myelinating oligodendrocyte markers. Use of these selectable markers resulted in highly specific selection of an oligodendrocyte precursor which differentiates into mature oligodendrocytes when the immortalizing gene is inactivated (in this case, by shifting the temperature at which the cells were being maintained to the nonpermissive temperature, 39°C). Construction of this cell line is described in Example 3. An oligodendrocyte optic nerve cell line (ts U195) was deposited (June 1, 1988) at the American Type Culture Collection (Rockville, MD), under the terms of the Budapest Treaty, and has been assigned accession number ATCC CRL9729.

A cell line of this type is potentially useful in replacement therapy in dysmyelinating diseases. Oligodendrocytes produced according to the present method can be assessed for their effectiveness in reversing (partially or completely) the effects of a genetic defect which normally results in debilitation and death in animals in which the defect occurs. Murine mutants which have defective myelination in the central nervous system and/or the peripheral nervous system, can be used to investigate the effectiveness replacement therapy with conditionally immortalized precursor cell lines. Jaque, C. et al., Journal of Neurochemistry, 41:1335-1340 (1983). Survival of the animals or less severe effects of the dysmyelination will be evidence of correction of the defect.

Cell lines of the present invention, in which growth promoting gene expression is regulated, differ from currently available cell lines in which the immortalizing agent which establishes the cell line is not normally under external control. It is possible with this new technique to obtain proliferating cell lines which can be grown rapidly to large numbers and then cause these cells to become a differentiated derivative. Until development of the present method, it was difficult or impossible to obtain such derivatives (e.g., because they grow much less rapidly or do not grow at all).

The method of the present invention provides a means by which large numbers of differentiated cells can be produced. Cells conditionally immortalized by the method of the present invention have diagnostic and therapeutic applications. For example, if a large quantity of a specific type of differentiated cell is needed for use in diagnostic methods, for transplantation into an individual, or for use as a means of producing a desired product (e.g., protein, hormone, etc.), appropriate precursor (nondifferentiated) cells can be selected, using known techniques. The progenitor cells can subsequently be modified by introduction of a growth promoting gene, such as an oncogene, then cultured under conditions in which the growth promoting gene is activated. When differentiated cells are needed, the cells are switched to conditions under which the growth promoting gene is inactivated. The previously undifferentiated cells are thereby altered, producing the desired differentiated cells.

Alternatively, conditionally immortalized cells can be introduced (at a nonpermissive temperature) into an individual, in whom they will reside at the nonpermissive (i.e., body) temperature. The conditionally immortalized cells will then differentiate, thus providing an "internal" source of the desired type of differentiated cell.

Recent work on central nervous system (CNS) transplants has been exciting and suggests rational approaches to therapy for CNS injury. Sladek, J.R. and D.M. Gash, Neural Transplants: Development and Function, Plenum Press, N.Y. (1984). The work described herein is directly relevant to transplant studies. Methods developed to identify and count cells will be useful in analyzing the fate of transplanted cells or the behavior of cells after injury (K. Frederiksen and R.D.G. McKay, 1988). The method disclosed in this invention which generates immortal cell lines that differentiate in vivo should be very important for cell replacement therapies.

Human JC virus infection is associated with the demyelinating disease, progressive multifocal leucoencapalopathy (PML). (ZuRhein, G.M., Prog. Med. Virol., 11:185-206 (1969); Padgett et al., Lancet, i:1257-1259 (1971); Padgett, B.L. and D.L. Walker, Prog. Med. Virol., 22:1-34 (1976)). Transgenic mice have been developed which express the early gene of the JC papovavirus associated with PML. These animals fail to myelinate their CNS (Small et al., 1986). This important finding shows that the whole course of viral infection is not necessary to cause disease - JC early gene expression alone is sufficient. In JC virus infection early gene (T antigen)-mediated disruption of oligodendrocyte differentiation may occur either by the action of T antigen directly in oligodendrocyte precursors or indirectly in another cell type which regulates oligodendrocyte differentiation.

Type 1 astrocytes are known to play an important role in the differentiation of oligodendrocyte precursors and are therefore candidates for the site of action of T antigen function in causing PML. Noble, M. and K. Murray, EMBO Journal, 3:2243-2247 (1984); Raff et al., Cell 42:61-69 (1985). Retroviral vectors expressing papovavirus T antigens have been shown capable of immortalizing astrocytic precursors. Immortalized astrocyte precursors can be used to produce large numbers of differentiated astrocytes and, thus, used in the production of oligodendrocytes.

Multiple sclerosis, while not clearly associated with a virus, disturbs the differentiation of oligodendrocytes (McKhann, G.M., Ann. Rev. Neurosci., 5:219-239 (1982). The ability to infect and immortalize cells of the early nervous system with papovavirus T antigens provides an unusual opportunity to manipulate cell types which interact to control oligodendrocyte differentiation.

Many neural tumors are histologically related to cells found in the early nervous system (Rubinstein, L.J., J. Neurosurg., 62:795-805 (1985). It is therefore crucial to characterize the factors controlling the differentiation of neural cells and to determine how oncogenes disrupt differentiation. This can be accomplished using the method and conditionally immortalized cells of the present invention.

The present invention will now be illustrated by the following Examples, which are not to be seen as limiting in any way.

### EXAMPLE 1 Construction of retroviruses carrying oncogenes and a dominant selectable marker

#### pZipSV40tsA58

The recombinant retrovirus pZipSV40tsA58 was constructed by the insertion of the BglI - HpaI fragment of the temperature sensitive SV40 tsA58 early region into the BamHI site of the pZipNeoSV shuttle vector in the sense orientation with respect to viral transcription. Cepko, C. et al., Cell, 37:1053-1062 (1984); Jat, P.S. et al., Molecular and Cellular Biology, 6:1204-1217 (1986); Jat, P.S. and P.A. Sharp, Journal of Virology, 59:746-750 (1986), the teachings of which are incorporated herein by reference. This SV40 fragment lacks both a promoter and polyadenylation sites. Consequently, transcription of T antigen sequences is exclusively initiated in the viral LTR. The recombinant plasmid was transfected into the psi 2 helper cell line and neomycin resistant cell lines were derived by G418 selection. Mann, R. et al., Cell, 33:153-159 (1983), the teachings of which are incorporated herein by reference. These transfected cell lines secrete defective helper-free recombinant retroviruses carrying the thermolabile T antigen gene. The titre of recombinant virus was determined by infection of 3T3 cells. By growing these 3T3 cell lines at 33° and 39°C, it was determined by immunohistochemistry and immunoprecipitation that they synthesize a ts form of SV40 T antigen. Culture medium from one of the highest titre psi 2 lines, psi2SVtsA58-4 ($10^4$ neo.resistant 3T3 units/ml.), was the source of virus for the infection of primary cultures of rat neural cells.

The target cells for viral infection were derived from the E18 entorhinal cortex and the P 2 cerebellum. The cells were dissociated by incubation in PBS, 0.5 mm EDTA, 0.25% trypsin, at 37°C for 20-30 minutes. The trypsin solution was removed and replaced with DMEM, 10% fetal bovine serum. The cells were triturated by 10-20 passages through a narrow bore pasteur pipette. The cell suspension was plated onto polyornithine coated tissue culture dishes at a density of $10^5$ cells/cm$^2$. Cell survival was greater than 70%, as determined by fluorescein diacetate uptake. Several schedules were examined for the optimal time of infection from infection in suspension, from 0 hrs. to 1 week post dissection. 4-12 hours after plating was found to yield the largest number of neo resistant colonies. Infection was achieved by replacing the culture medium on the attached cells with a small volume (0.8 ml./10 cm. dish) of sterile medium from the producer line psi2SVtsA58-4 containing 8 microgm./ml. of polybrene. The cells were incubated for 2 hours at 37°C. 8-10 volumes of 50-50 medium were then added and the cells plated at 33°C. The medium was changed the following day and four days postinfection, when selection for infected, neo resistant cells was initiated by the addition 0.2 mg./ml. G418 SO$_4$ (Geneticin, GIBCO). Cell death became apparent after 1 week of selection. Living colonies remained present in uninfected controls for as long as 1 month after the application of G418 selection. Resistant colonies were picked when they contained approximately 1000 cells. In general, primary embryonic brain cultures gave ten to thirty fold fewer colonies than a similar number of NIH 3T3 cells.

Cultures were passaged at high density and fed twice weekly. The 50/50 growth medium contained DMEM, 5% F.C.S., 2.5 microg./ml. insulin, 10 nm. progesterone, 10 pm. beta-estradiol, 1 microg./ml. transferrin, 0.1 mm. putrescine, 30 nm. sodium selenite, 0.3 nm. triiodothyronine, 20 nm. hydrocortisone, 2 mm. sodium pyruvate.

Other retroviruses can also be constructed, as described below.

#### Construction of pZipSV40 (U19) tsA58

Paucha et al. described a variant of SV40 T antigen (U19) which was deficient in certain viral functions, but has recently been shown to have enhanced ability to immortalize primary fibroblasts. Paucha, E. et al., Journal of Virology, 57:50-64 (1986); Jat, P.S. et al., Molecular and Cellular Biology, 6:1204-1217 (1986), the teachings of which are incorporated herein by reference. The U19 and tsA58 mutations fall in different parts of large T antigen. A retrovirus double mutant with both increased immortalization efficiency and thermolability has been constructed, as described in Example 3.

pZipPytsA (1T)

Because cell culture suggests that the polyoma large T-antigen has a less powerful effect on the immortalized cells than SV40 T antigen, a retrovirus carrying the transforming early gene of polyoma virus can also be constructed and should be useful. Polyoma virus large T antigen immortalized primary fibroblasts are not transformed by supertransduction with the ras oncogene. In contrast, the ras oncogene will transform primary fibroblasts that have been immortalized with the SV40 T antigen. This result shows that polyoma and SV40 large T antigens interact with primary fibroblasts in different ways. One interpretation is that polyoma large T is 'milder' and this mild effect may be an advantage from the point of view of using the oncogenes for precursor cell immortalization. A temperature sensitive variant of mouse polyoma large T antigen is available. Fried, M., Proceedings of the National Academy of Sciences, USA, 53:486-542 (1965).

Unlike SV40 early region, the early region of polyoma virus encodes two proteins with oncogenic properties. The middle T protein is transforming and the large T protein immortalizing. A useful retrovirus will contain only the sequences necessary for the large T antigen and the tsA form of this protein. A plasmid carrying the wild type large T antigen cDNA and a second plasmid carrying the entire tsA early region have been constructed and generously provided by Dr. R. Kamen (Genetics Inst.). The region carrying the altered DNA sequences which confer thermolability can be placed into the large T antigen cDNA and a retrovirus constructed.

pZipJCtsA

Evidence from transgenic mice shows that the human polyoma virus T antigen disrupts the differentiation of oligodendrocytes and may account for the demyelination found in the disease progressive multifocal leucoencapalopathy (PML). Papovavirus oncogenes, such as those derived from the three closely related viruses mouse polyoma, monkey SV40 and human JC can be used to construct a retrovirus. For example, the polyoma and SV40 thermolabile sequences can be placed onto the JC virus large T antigen.

EXAMPLE 2 Immortalization of Cerebellar Cells

Three different oncogenes, SV40 T antigen, v-myc and neu were tested for their ability to establish cell lines from the developing cerebellum. Materials and procedures used were as follows:

Markers used to identify precursor populations

Prior to establishing cell lines from the postnatal cerebellum, the proliferating cells which are potential targets for retroviral mediated gene transfer were identified. Three classes of antibody were used as markers to define the proliferative status of different cell types in the postnatal cerebellum: the monoclonal antibody Rat 401, anti-vimentin and anti-GFAP antibodies.

The monoclonal antibody Rat 401 recognizes a transient population of embryonic columnar epithelial cells and radial glial cells in many regions of the rat CNS. Hockfield, S. and R. McKay, Journal of Neuroscience, 12:3310-3328 (1985). The antibody recognizes a 200 Kd. protein and some smaller peptides which are variably found and may be degradation products. Known methods of measuring total cell numbers in the rat CNS during development were used with immunohistochemistry and techniques for labelling proliferating cells to show that Rat 401 recognized a major population of proliferating neuronal precursor cells in the spinal cord. Frederiksen, K. and R. McKay, J. Neurosci. (1988). In primary culture of embryonic brain cells a transient co-expression of the Rat 401 antigen and either neurofilaments or the astrocytic intermediate filament protein, GFAP, was seen in morphologically distinct cell types. These data suggest that the Rat 401 positive population contains precursors to both neurons and astrocytes. It has previously been shown in the cerebellum that the monoclonal antibody Rat 401 recognized a transient cell population. Hockfield, S. and R. McKay, J. Neurosci., 5:3310-3328 (1985). These studies support the use of the Rat 401 antigen as an assay for precursor cell lines.

Vimentin is found in the precursor cells to the cerebellar granule neurons (Bovolenta, P. et al., Dev. Biol., 102:248-259 (1984) as well as neuronal and glial precursor cells elsewhere in the nervous system. Vimentin is not generally found in mature neurons and astrocytes in vivo. About the time neurons become postmitotic, they begin to synthesize a specific set of intermediate filament proteins, neurofilaments, and lose vimentin expression.

6

Glial fibrilary acidic protein (GFAP) is the core intermediate filament protein which replaces vimentin in astrocytes (reviewed in Fields, 1985). This protein is expressed in the adult cerebellum in the radial Bergman glial cells and in astrocytes. GFAP is also found in some cells of the germinal zone of the monkey neuroepithelium during neurogenesis (Levitt, P. et al., J. Neurosci., 1:27-39 (1981). These immunohistochemical observations and our quantitation of cell populations in vivo (Frederiksen, K. and R. McKay, J. Neurosci., (1988)) suggested that there was a proliferating glial precursor cell which was Rat 401 negative and GFAP positive.

As Rat 401 antigen, vimentin and GFAP are potential markers for distinct precursor populations, the differential expression of these antigens was used to characterize cell populations in the postnatal cerebellum. Dissociated cerebellar cells were stained with Rat 401, anti-vimentin and anti-GFAP antibodies. The proportion of Rat 401 positive cells declines to zero before postnatal day 15 (P15), showing that Rat 401 is a precursor cell marker and confirming previous immunohistochemical analysis of cell differentiation in the postnatal cerebellum. The proportion of vimentin positive cells is much larger than the proportion of Rat 401 positive cells but also declines over this period. The proportion of GFAP positive cells is initially lower than the proportion of Rat 401 positive cells but by the end of the second postnatal week the number of GFAP positive cells increases while the expression of Rat 401 is lost.

These three markers are differentially expressed in cerebellar cell populations but further data is needed to establish if these cell populations are independent of one another and actively proliferating. The overlap in cell populations was determined by double label immunohistochemistry (Table 1) and the proliferative status of the antigenically distinct cell types was measured by an in vivo pulse of tritiated thymidine followed by immunohistochemistry and autoradiography on dissociated cells (Table 2).

Double immunohistochemistry showed that the Rat 401 positive population was a subset of the vimentin population but the autoradiographic data shows that these two populations are not in identical proliferative states. The GFAP positive population and the vimentin positive populations also overlap.

## Animals, cell lines and antibodies

Sprague Dawley rats were obtained from Taconic Inc., N.Y. NIH 3T3 cells and psi2 cells lines were grown in Dulbecco's modified Eagle's medium (DMEM) with 10% (v/v) calf serum, penicillin and streptomycin. The anti-vimentin antibody was obtained from ICN (Cat. No. 69-127), the anti-GFAP antibody was obtained from ICN (Cat- No- 69-110), and the anti-neurofilament antibody was Sternberger-Meyer (Cat. No. SMI 31), the Rat 401 antibody was established in this group and has been previously described (Hockfield, S. and R. McKay, J. Neurosci., 5:3310-3328 (1985)), the anti-T antibody was monoclonal antibody 412 prepared by E. Harlow, the anti-Gal C antibody has been previously described Ranscht et al., 1982. In double label experiments rabbit anti-vimentin was obtained from R. Hynes, the rabbit anti GFAP from L. Eng., the rabbit anti-neurofilament was purchased from ICN (Cat. No. 20074). Secondary antibodies were obtained from Cappel-Worthington.

## Analysis of cerebellar cells types in vivo

A detailed description of dissociation protocols, autoradiographic procedures and control experiments for cell numbers and staining procedures can be found in Frederiksen, K. and R. McKay, J. Neurosci. (1988). The cerebellum was dissected from postnatal rats and the cells dissociated by trituration after digestion in 0.15% trypsin in $Ca^{++}$ and $Mg^{++}$ free phosphate buffered saline. Measured aliquots of the dissociated cell suspension were spun onto coverslips and stained with primary antibodies and either fluorescein, rhodamine or peroxidase conjugated second antibodies. The data shown was derived from four or more animals from two or more litters.

## Primary cultures and infection protocols

The cerebellum was removed from P2 animals and incubated in 0.08% trypsin for 30 minutes at 37°C. After further dissection into small pieces the cells were dissociated by trituration in DMEM, 10% fetal calf serum using a 20 microlitre pipetteman. The cell suspension was plated onto polyornithine coated tissue culture dishes (15 micrograms/ml, Sigma) in DMEM, 10% fetal calf serum and incubated at 37°C 24 hours after plating the cells were infected for 2 hours with the recombinant retroviruses in 8 micrograms/ml (Aldrich). After infection the virus containing medium was replaced with fresh DMEM, 10% fetal calf serum. tsA58 infected cell lines were subsequently grown at 33°C. 48 hours after infection the cultures were passaged and subjected to selection in 0.2 micrograms/ml G418 (Geneticin, Gibco). The medium was changed with fresh G418 every 3-4 days. Within three weeks control uninfected dishes had very few remaining cells and the infected dishes had macroscopic G418 resistant colonies. Colonies were picked using cloning rings and expanded into 96 well plates.

Growth and characterization of cell lines

The cells were continually grown in DMEM and 10% fetal calf serum. The neu infected cells could be expanded and frozen but always grew slowly. The v-myc and T-antigen derived cells grew rapidly for a year and were subcloned. The immunohistochemical analysis of antigen expression in St15A cells was carried out in DMEM and selected batches of fetal calf serum at 33°C and 39°C. The immunoblotting procedure was as described by Twobin et al. (1979). Analysis of proviral DNA was carried out on high molecular weight DNA prepared by the method Shih and Weinberg (1982) and fractionated on 0.8% agarose gels. The DNA was transferred to Zeta Bind (CUNO Labs, Meriden, Conn.) and hybridized by standard methods. Southern, 1975; Maniatis et al., Molecular Cloning: A Laboratory Manual, Cold Spring Harbor Laboratory, Cold Spring Harbor, N.Y. (1982). The serum free medium used was the N2 medium of Bottenstein and Sato (1979). The co-cultures were carried out after labelling the immortal cells with either fluorescein succinimidylester (Bronner-Fraser, M., Journal of Cellular Biology, 101:610 (1985)), the carbocyanine dye diI (Honig, M.G. and R.I. Hume, Journal of Cellular Biology, 103:171-187 (1986)) or rhodaminated latex beads (Katz et al., 1984). These labelled cells were then added to primary cultures of E14 cerebral cortex or P2 cerebellum one day after the primary cells were plated. The double label experiments with Rat 401 and rabbit anti-neurofilament antibody used the cross reaction between anti-MSH antibody and neurofilament analyzed by Verhaagen et al. (1986).

v-myc and SV40 T antigen have previously been shown to be efficient in establishing rodent fibroblast cell lines. Land, H. et al., Nature, 304:596-602 (1983); Jat and Sharp, Molecular and Cellular Biology, 6:1204-1217 (1986). In contrast, the neu oncogene has not previously been shown to establish primary rodent cell lines. However, activated neu is associated with neuroblastomas and glioblastomas (Schechter, A.L. et al., Nature, 312:513-516 (1984)) and the cellular neu is known to be transcribed and translated in the early nervous system. SV40 virus has been shown to generate cell lines with either neuronal or glial properties (DeVitry, F. et al., Proceedings of the National Academy of Sciences, USA, 77:4165-4169 (1974); Neto, V.M. et al., Dev. Brain Res., 26:11-22 (1986)).

Details of the construction of the v-myc transducing retrovirus can be found elsewhere (Dotto et al., 1986). The neu transducing retrovirus was a generous gift of Dr. C.I. Bargmann. SV40 T antigen has another advantage in that it is available in a tight temperature sensitive form, making it possible to switch off the function of the oncogene by raising the temperature of cell growth. Tegtmeyer, P., Journal of Virology, 15:613-618 (1985); Tegtmeyer, P., In: J. Tooze (ed.), Molecular Biology of Tumor Viruses (2d ed.), Cold Spring Harbor Laboratory, pp. 297-338 (1980). A transducing retrovirus carrying the tsA58 mutant form of SV40 large T antigen was constructed (Figure 1 and Example 1). The ability to externally regulate the function of the growth promoting gene makes it possible to determine if a precursor cell line can differentiate in the absence of the immortalizing gene function.

The three recombinant retroviruses described were all constructed by inserting DNA sequences encoding the oncogenes into the unique BamHI site of the pZipNeoSV(X)I vector (Figure 1); the genes were inserted in the sense orientation with respect to retroviral transcription driven by the cis-acting transcriptional regulatory sequences in the left-hand long terminal repeat (LTR). Previous work has shown that different regulatory sequences allow the same oncogene to interact with different cell types. Because the only differences in the three retroviruses used here were in the coding sequences, any differences in the derived cell lines must be due to the oncogenes themselves and not to the regulatory sequences controlling transcription. All the pZipNeoSVtsA58 producer cell lines were shown to synthesize virus which only transduce large T antigen. Thus the reported enhancement of transformation by SV40 small T antigen (Bikel et al., 1987) is not a complicating factor here.

Properties of Cerebellar Cell Lines

To be a candidate for immortalization, a cell type must be both present and proliferating in primary culture. The antigenic and proliferative status of P2 cerebellar cells in dissociated tissue culture was measured by immunohistochemistry and thymidine autoradiography. Rat 401 positive cells are present in short term primary culture. The number of cells expressing GFAP is very low. The immunohistochemical data also shows that vimentin positive cells are present in primary culture. Antigenic and autoradiographic double label showed that Rat 401 positive and vimentin positive cells proliferate in primary culture but the GFAP positive cells are quiescent.

After infection and selection, isolated colonies were picked. Cell lines were characterized immunohistochemically with Rat 401, anti-GFAP and anti-vimentin antibodies. As shown in Table 3, all but one of the derived cell lines were Rat 401 positive and all were vimentin positive. GFAP expression was found in all of the cell lines derived from myc and neu infection; in contrast the SV40 immortalized cell lines were GFAP negative at the permissive temperature. These results show that it is possible to obtain cell lines expressing markers characteristic of three proliferating populations defined in Tables 2 and 3.

# TABLES

## Table 1.

Proportion of Rat 401 and GFAP positive P3 cerebellar cells double labelled with other antibodies.

| Rat 401 +, | 96 % | vimentin + |
| Rat 401 +, | 53 % | GFAP + |
| GFAP +, | 77 % | Rat 401 + |
| GFAP +, | 80 % | vimentin + |

## Table 2.

Proportion of antibody labelled cells which are also radiolabelled by an in vivo pulse of tritiated thymidine two hours before cell dissociation.

| Antibody | % cells radiolabelled on postnatal day | | | | |
|---|---|---|---|---|---|
| | 2 | 7 | 9 | 12 | 15 |
| Rat 401 | 8 | 8 | 9 | - | - |
| vimentin | 17 | 16 | 20 | 21 | 25 |
| GFAP | 9 | 11 | 10 | 6 | 5 |

## Table 3.

Antigenic profile of cerebellar cell lines.

| Immortalizing gene | No. of lines | Primary antibodies | | |
|---|---|---|---|---|
| | | Rat 401 | Vimentin | GFAP |
| SV40tsA58 | 9 | + | + | - |
| | 1 | - | + | - |
| v-myc | 31 | + | + | + |
| neu | 4 | + | + | + |

Two of the SV40 cell lines were grown at 33°C and 39°C and immunohistochemically analyzed for differentiation. The ST15I cell line was Rat 401 negative and did not differentiate at 39°C in fetal calf serum. The ST15A line was used as a representative of the Rat 401 positive class. The results for AT15A show, as expected, that at the nonpermissive temperature, the cells lose T antigen expression. At the elevated temperature, these cells also lose Rat 401 antigenicity and gain GFAP expression. This antigenic switch in the cells is remarkably complete. At 33°C Rat 401 negative cells or GFAP positive cells occur with a frequency of less than $10^3$ at 33°C. After several days at 39°C, the majority (95%) of the ST15A cells were Rat 401 negative and GFAP positive. An increase in GFAP expression was confirmed by immunoblotting of total proteins extracted from cells grown at the permissive and nonpermissive temperature. Immunoblotting also showed that T antigen levels fall at the non-permissive temperature. The large cultures necessary for protein chemistry differed from the analytical cultures used for the immunohistochemical analysis as Rat 401 expression remained in 25% of the cells after 10 days of growth at the elevated temperature. Vimentin levels were unchanged.

ST15A were grown in the N2 medium of Bottenstein and Sata (1979). After 5 days at 39°C, cells with a neuronal morphology, which react with the antineurofilament monoclonal antibody SMI 31, were seen. After nine days in N2 medium, the proportion of SMI 31 positive cells increased (to 90% in some cultures) and cells with very long neurites (greater than 300 microns) were present. Cells with this highly distinctive neuronal morphology were present both at 33°C and 39°C.

When live ST15A cells grown in 10% fetal calf serum were stained with an anti-galactocereberoside monoclonal antibody, approximately 1% of the cells were positive. The labelling of live cells and the distribution of the antigen shows that this is a surface component that is recognized by the anti-gal C antibody. The frequency of anti-GalC labelled cells was enhanced when ST15A cells were grown at the non-permissive temperatures. Galactocereberoside expression has been extensively used as a marker for commitment to oligodendrocyte differentiation (Raff, M.C. et al., Nature, 274:813-186 (1978), 1979; Schachner, M. and M. Willinger, In: The Menarini Series of Immunopathology, Vol. 2, pp. 37-60 (1979).

As the mechanisms controlling the differentiation of the neuroepithelium depend on cell interaction, differentiation of tsA cerebellar cell lines upon co-cultivation with primary embryonic brain cells was assessed. To identify the clonal immortal cells in the presence of primary cells, they were first labeled internally with either the succinimidyl ester of fluorescein (Bronner-Fraser, M., Journal of Cellular Biology, 101:610 (1985)) or the lipid soluble carbocyanine dye diI (Honig, M.G. and R.I. Hume, Journal of Cellular Biology, 103:171-187 (1986)). These internally labelled cells were then analyzed after they were added to primary rat cultures. The primary cells were derived from either the E14 cerebral cortex or from postnatal day 3 cerebellum. The mixed cultures were processed immunohistochemically with anti-T, anti-neurofilament, anti-vimentin, anti-GFAP, Rat 401 and the appropriate second antibody, tsA immortalized cells were identified by the internal label and the binding of antibody established in the same cell.

The initial experiments using this paradigm were carried out at 33°C because the red nuclear T antigen fluoroescence serves as a control for the internal labelling method. No internally labelled T antigen negative cells were seen. Confidence in these results is high because for each antibody there are large numbers of primary cells which are not internally labelled which acts as controls for the specificity of antibody staining.

In the presence of primary brain cells ST15A, cells can lose vimentin and Rat 401 antigenicity and gain either GFAP or neurofilament reactivity. Three different lines of evidence suggest that ST15I can differentiate into neurons: Firstly, many of the fluoroscein labelled cells adopt a morphology of small cell bodies with multiple processes. Secondly, like primary neurons these small ST15I cells are often found in clumps over flat non-neuronal cells. Thirdly, the processes of these small ST15I cells can be stained with an anti-neurofilament monoclonal antibody. ST15I cells which express the Rat 401 antigen were also found in co-culture. In the Rat 401 positive ST15I cells shown, the antigen is restricted to a small perinuclear region. This distribution is also characteristically found in primary neuroepithelial cells as they differentiate from the Rat 401 positive precursor state to the neurofilament positive differentiated neuron.

Cells with neuronal properties were also produced when the v-myc induced cell line M15B was treated with dibutryl cyclic AMP and retinoic acid. This induction is not complete, but characteristically occurs in a colonial manner, suggesting that a commitment step occurs in a proliferating cell which subsequently differentiates into neurons or that local interactions are important in neuronal induction.

Southern blot analysis was used to determine whether the cell lines were clonal, panel A was hybridized with SV40 probe and panel B with neomycin probe. The SV40 sequences were released from flanking DNA by Bam HI digestion yielding a 2.3 Kb fragment designated b. The size of this fragment is too small to encode the small t antigen transcript and confirms the immunohistochemistry and immunoblotting data, showing that the pZipSVtsA58 retrovirus transduces large T antigen. Xba cuts in the viral LTR sequences releasing a single full length proviral sequence from ST15A and ST15I. EcoRI and Bgl II digestion cuts once in the provirus, yielding only a single fragment in ST15A and ST15I containing SV40 sequences and flanking cellular DNA. This data demonstrates that the ST15A and ST15I cells carried only a single insertion. In contrast, the M15b cells showed two large neomycin complementary bands. These two bands are found in the same ratios in multiple subclones of M15B suggesting that the M15B cell line as two independent viral insertion sites and is also a clonal cell line.

The present data suggests that the use of the tsA58 variant of SV40 T antigen favors the differentiation of the multipotential brain precursor cell at the nonpermissive temperature of 39°C. The core body temperature of the rat is 39°C, suggesting that ts cell lines may differentiate when transplanted into the developing CNS. Wild type T immortalized cells have been transplanted into the immediately postnatal CNS of the rat. Results showed that as many as one million cells can be placed in the CNS, where they remain for several months without any gross indication of tumor formation (McKay et al., 1988). Transplantation of conditionally immortalized cell lines into the developing nervous system offers the most rigorous assay for the differentiation potential of these cell lines. Transplantation of primary neuroepithelium into the lesioned adult CNS has been used to restore behavioral function to lesioned animals. It may be possible to use cell lines in place of primary cells allowing a detailed analysis of the molecular genetics of behavioral recovery.

Example 3 Infection of optic nerve primary cultures with retrovirus ts U19

1. Optic nerve from 4-6 rats (P4) were dissociated according to the following protocol:

i) The nerves were placed in 2 ml of Basal Eagles Medium (BEM) with 0.02M Hepes (Sigma, T5391) and collagenase 0.02% (Sigma, type IV, C5138) for 15 minutes at 37°C.

ii) The BEM was then removed and 2 ml of HBSS, Cs$^{++}$ and Mg$^{++}$ "free" and 0.25% trypsin were added for 15 minutes at 37°C (40X, Sigma TO511)

iii) Cells were checked to determine whether they had begun to dissociate. Step i) and ii) were repeated, as required.

iv) 1 ml of media was removed and 1 ml of Dulbecco's Modified Eagles Medium (DMEM).10%FCS was added. The nerves were panned up and down 3-5X through an 18 gauge needle with a 2 ml syringe. This was repeated three times through a 21 gauge needle.

v) Large pieces of nerve were removed and the cell suspension was centrifuged to remove the digestion enzyme.

vi) The pellet was resuspended in DMEM with 10% fetal calf serum (FCS) and were plated on polyornithine-covered dishes.

2. One or two days after dissociation, the medium was replaced with psi 2 supernatant (producer cells). 2 ml of medium ($10^4$-$10^5$ cfu/ml.) were added to each 2.5 cm dish. Polybrene (Sigma, P4S15) was also added to each dish, to a final concentration of 8 ug/ml. Cells were incubated at 33°C for 2-3 hrs.

3. The virus-containing medium was removed and replaced with 2.5 ml of BMEM/10% FCS (same as in Step 1 vi).

4. Two days later, the infected cells were split 1:2 to 1:5. At this time G418 (Geneticin, Sigma G9516) was added to 200 micrograms/ml).

5. The medium was changed every 4.5 days and G418 was kept at the same concentration. Colonies began to form between 2-3 weeks later.

Cloning cell lines

1. 24 well petri dishes were treated with pOrn.

2. Cloning rings (glass, 7 mm θ and about 7 mm height) were placed with high vacuum gresse (Down Corning from Dupont) on a colony and 30-100 lambda of 1X trypsin/EDTA was added. Detachment of cells was checked under a microscope and cells were dissociated with 200 lambda pipetor and transferred to 24 wells/dish.

3. The clones were then assayed for the markers described previously.

Construction of plasmid (pZip tsa 58 U19 or pts U19)

The plasmid pZiptsa58U19 or ptsU19 is represented in Figure 2. U19 is a variant of SV40 T antigen, described by Paucha and co-workers. Paucha, E. et al., Journal of Virology, 57:50-64 (1986). The U19 and tsA58 mutations occur in different parts of the large T antigen; Paucha et al. describe mutation of the large T antigen sequence from serine (amino acid position 152) and arginine (amino acid position 154) to, respectively, asparagine and lysine in the U19 mutant.

1. The tsA58 temperature sensitive form of the SV40 T antigen was placed in the pZipNeo SV (X)1 vector by inserting the whole early region (BglI-HpaI) of tsA58 into the Bam HI site.

2. The hybrid of the SV40 T antigen gene, tsU19, was constructed using the pZip tsA58 and the U19 mutant.

Equivalents

Those skilled in the art will recognize, or be able to ascertain using no more than routine experimentation, many equivalents to the specific embodiments of the invention described specifically herein. Such equivalents are intended to be encompassed in the scope of the following claims.

## Claims

1. A method of producing differentiated neural cells, comprising culturing appropriate neural precursor cells, having incorporated therein a growth-promoting gene whose activation or inactivation is controlled by an external factor, under conditions which activate said growth-promoting gene; maintaining said precursor cells under appropriate conditions and for sufficient time for growth of said precursor cells; and inactivating said growth-promoting gene by shifting to conditions which inactivate said growth-promoting gene.

2. Differentiated neural cells obtainable by the method of Claim 1.

3. A method of immortalizing a neural precursor cell line, to produce differentiated neural cells, comprising:
   a. introducing into appropriately-selected neural precursor cells in primary culture, under permissive conditions, a growth-promoting gene which is activated under said permissive conditions, said growth-promoting gene which is activated under said permissive conditions, said growth-promoting gene conferring on said neural precursor cells the ability to grow in tissue culture, to produce neural cells containing said growth-promoting gene;
   b. selecting neural cells containing said growth-promoting gene and maintaining them under permissive conditions and in medium appropriate for neural cell growth, for sufficient time for neural cell growth;
   c. shifting conditions in (b) to nonpermissive conditions, to inactive said growth promoting gene, and maintining the neural cells under conditions appropriate for neural cell growth and differentiation to occur.

4. Differentiated neural cells obtainable by the method of Claim 3.

5. A method of producing differentiated neural cells, comprising:
   a. introducing into appropriately-selected neural precursor cells a retroviral vector comprising a growth-promoting gene which is the temperature-sensitive domain of the tsA58 strain of the SV40 virus;
   b. culturing neural cells produced in (a) at a temperature of approximately 33°C and under conditions sufficient for cell growth;
   c. shifting the temperature of the cultured neural cells to approximately 39°C; and
   d. maintaining the neural cells under conditions appropriate for and for sufficient time for cell differentiation to occur.

6. Differentiated neural cells obtainable by the method of Claim 5.

7. A method of immortalizing neural cells from the embryonic nervous system, comprising:
   a. selecting appropriate neural precursor cells;
   b. introducing into said neural precursor cells in primary culture a retrovirus comprising the temperature sensitive domain of the tsA58 strain of the SV40 virus and a gene encoding drug resistance;
   c. selecting drug resistant cells and maintaining them under conditions appropriate for cell growth and at a temperature of approximately 33°C, for sufficient time for cell growth to occur; and
   d. shifting the temperature to approximately 39°C.

8. The method of Claim 7 wherein the neural precursor cells are derived from embryonic hippocampus or from embryonic cerebellum.

9. An immortalized neural cell line obtainable by incorporating into appropriate neural precursor cells a growth-promoting gene which enables the neural cells to grow and is controlled by an external factor.

10. An immortalized neural cell line obtainable by incorporating into embryonic nervous system cells a retroviral vector comprising a growth-producing gene which is the temperature-sensitive domain of the tsA58 strain of the SV40 virus and a gene encoding drug resistance, culturing said cells at a temperature of approximately 33°C under conditions sufficient for and for sufficient time for cell growth to occur and subsequently culturing said cells under appropriate conditions and at a temperature of approximately 39°C.

11. The oligodendrocyte optic nerve cell line, ts U195, deposited at the American Type Culture Collection under accession number CRL9729.

EP 0 428 519 B1

**Patentansprüche**

1. Verfahren zur Herstellung von differenzierten neuralen Zellen, wobei das Verfahren folgende Schritte aufweist: Züchten von geeigneten neuralen Vorläuferzellen, in die ein wachstumsförderndes Gen eingebaut ist, dessen Aktivierung oder Inaktivierung durch einen externen Faktor gesteuert wird, unter Bedingungen, die das wachstumsfördernde Gen aktivieren; Halten dieser Vorläuferzellen unter geeigneten Bedingungen und über einen ausreichenden Zeitraum für das Wachstum der Vorläuferzellen; und Inaktivieren des wachstumsfördernden Gens durch Verschieben zu Bedingungen, die das wachstumsfördernde Gen inaktivieren.

2. Differenzierte neurale Zellen, die mit dem Verfahren gemäß Anspruch 1 zu erhalten sind.

3. Verfahren zum Unsterblichmachen einer neuralen Vorläuferzellinie, um differenzierte neurale Zellen zu erzeugen, wobei das Verfahren folgende Schritte aufweist:
   a. Einbringen eines wachstumsfördernden Gens in geeignet ausgewählte neurale Vorläuferzellen in primärer Kultur unter permissiven Bedingungen, wobei das wachstumsfördernde Gen unter den permissiven Bedingungen aktiviert wird, wobei das wachstumsfördernde Gen den neuralen Vorläuferzellen die Fähigkeit verleiht, in Zellkultur zu wachsen, um neurale Zellen zu erzeugen, die das wachstumsfördernde Gen enthalten;
   b. Auswählen von neuralen Zellen, die das wachstumsfördernde Gen enthalten, und Halten derselben unter permissiven Bedingungen und in einem für das Wachstum neuraler Zellen geeigneten Medium über einen für das Wachstum neuraler Zellen ausreichenden Zeitraum;
   c. Verschieben der Bedingungen in (b) zu nichtpermissiven Bedingungen, um das wachstumsfördernde Gen zu inaktivieren, und Halten der neuralen Zellen unter Bedingungen, die geeignet sind, um das Auftreten von neuralem Zellwachstum und Differenzierung zuzulassen.

4. Differenzierte neurale Zellen, die mit dem Verfahren nach Anspruch 3 zu erhalten sind.

5. Verfahren zur Herstellung von differenzierten neuralen Zellen, wobei das Verfahren folgende Schritte aufweist:
   a. Einbringen eines retroviralen Vektors in geeignet ausgewählte neurale Vorläuferzellen, wobei der Vektor ein wachstumsförderndes Gen aufweist, das die temperatursensitive Domäne des tsA58-Stamms des SV40-Virus ist;
   b. Züchten von in (a) erzeugten neuralen Zellen bei einer Temperatur von ca. 33 °C und unter Bedingungen, die für Zellwachstum ausreichend sind;
   c. Verschiebenn der Temperatur der gezüchteten neuralen Zellen auf ca. 39 °C; und
   d. Halten der neuralen Zellen unter Bedingungen, die geeignet sind, und über einen Zeitraum, der ausreichend ist, um das Auftreten der Zelldifferenzierung zuzulassen.

6. Differenzierte neurale Zellen, die mit dem Verfahren nach Anspruch 5 zu erhalten sind.

7. Verfahren zum Unsterblichmachen von neuralen Zellen aus dem embryonalen Nervensystem, wobei das Verfahren folgende Schritte aufweist:
   a. Auswählen geeigneter neuraler Vorläuferzellen;
   b. Einbringen eines Retrovirus in die neuralen Vorläuferzellen in primärer Kultur, wobei das Retrovirus die temperaturempfindliche Domäne des tsA58-Stamms des SV40-Virus und ein für eine Stoff-Resistenz codierendes Gen aufweist;
   c. Auswählen von stoff-resistenten Zellen und Halten derselben unter Bedingungen, die für ein Zellwachstum geeignet sind, und bei einer Temperatur von ca. 33 °C für eine ausreichende Zeit, um das Auftreten von Zellwachstum zuzulassen; und
   d. Verschieben der Temperatur auf ca. 39 °C.

8. Verfahren nach Anspruch 7, wobei die neuralen Vorläuferzellen von embryonalem Hippocampus oder embryonalem Cerebellum abgeleitet sind.

9. Unsterblich gemachte neurale Zellinie, erhältlich durch Einbringen eines wachstumsfördernden Gens in geeignete neurale Vorläuferzellen, wobei das Gen das Wachstum der neuralen Zellen zuläßt und von einem externen Faktor gesteuert wird.

13

**10.** Unsterblich gemachte neurale Zellinie, die erhalten wird, indem in Zellen eines embryonalen Nervensystems ein retroviraler Vektor eingebracht wird, der ein wachstumsförderndes Gen, das die temperaturempfindliche Domäne des tsA58-Stamms des SV40-Virus ist, und ein für eine Stoff-Resistenz codierendes Gen aufweist, Züchten der Zellen bei einer Temperatur von ca. 33 °C unter Bedingungen, die geeignet sind, und über einen Zeitraum, der ausreichend ist, um das Auftreten von Zellwachstum zuzulassen, und anschließendes Züchten dieser Zellen unter geeigneten Bedingungen und bei einer Temperatur von ca. 39 °C.

**11.** Die Oligodendrocyt-Zellinie des Nervus opticus, ts U195, die bei der American Type Culture Collection unter der Eingangsnummer CRL9729 hinterlegt ist.

## Revendications

**1.** Méthode de production de cellules neurales différenciées, comprenant les opérations consistant à cultiver des cellules précurseuses neurales appropriées, dans lesquelles est incorporé un gène promoteur de croissance dont l'activation ou l'inactivation est régie par un facteur extérieur, dans des conditions qui activent ledit gène promoteur de croissance; à maintenir lesdites cellules précurseuses dans des conditions appropriées et pendant un temps suffisant pour la croissance desdites cellules précurseuses; et à inactiver ledit gène promoteur de croissance en passant à des conditions qui inactivent ledit gène promoteur de croissance.

**2.** Cellules neurales différenciées, pouvant être obtenues par la méthode selon la revendication 1.

**3.** Méthode d'immortalisation d'une lignée de cellules précurseuses neurales, pour produire des cellules neurales différenciées, comprenant les opérations consistant
a. à introduire dans des cellules précurseuses neurales convenablement sélectionnées en culture primaire, dans des conditions permissives, un gène promoteur de croissance qui est active dans lesdites conditions permissives, ledit gène promoteur de croissance donnant auxdites cellules précurseuses neurales la capacité de croître en culture tissulaire, pour produire des cellules neurales contenant ledit gène promoteur de croissance;
b. à sélectionner des cellules neurales contenant ledit gène promoteur de croissance et à les maintenir dans des conditions permissives et dans un milieu approprié pour la croissance de cellules neurales, pendant un temps suffisant pour la croissance de cellules neurales;
c. à passer des conditions établies en (b) à des conditions non permissives, pour inactiver ledit gène promoteur de croissance, et à maintenir les cellules neurales dans des conditions appropriées pour que se produisent la croissance et la différenciation des cellules neurales.

**4.** Cellules neurales différenciées, pouvant être obtenues par la méthode selon la revendication 3.

**5.** Méthode de production de cellules neurales différenciées, comprenant les opérations consistant
a. à introduire, dans des cellules précurseuses neurales convenablement sélectionnées, un vecteur rétroviral comprenant un gène promoteur de croissance qui est la région sensible à la température de la souche tsA58 du virus SV40;
b. à cultiver des cellules neurales produites en (a) à une température d'environ 33°C et dans des conditions adéquates pour la croissance des cellules;
c. à faire passer la température des cellules neurales cultivées à environ 39°C; et
d. à maintenir les cellules neurales dans des conditions appropriées et pendant un temps suffisant pour que la différenciation des cellules se produise.

**6.** Cellules neurales différenciées, pouvant être obtenues par la méthode selon la revendication 5.

**7.** Méthode d'immortalisation de cellules neurales provenant du système nerveux embryonnaire, comprenant les opérations consistant
a. à sélectionner des cellules précurseuses neurales;
b. à introduire dans lesdites cellules précurseuses neurales en culture primaire un rétrovirus comprenant la région sensible à la température de la souche tsA58 du virus SV40 et un gène codant pour la résistance à une drogue;
c. à sélectionner des cellules résistantes à la drogue et à les maintenir dans des conditions appropriées pour la croissance des cellules et à une température d'environ 33°C, pendant un temps suffisant pour que la croissance des cellules se produise; et

d. à faire passer la température à environ 39°C.

8. Méthode selon la revendication 7, dans laquelle les cellules précurseuses neurales sont dérivées d'hippocampe embryonnaire ou de cervelet embryonnaire.

9. Lignée cellulaire neurale immortalisée, pouvant être obtenue en incorporant, dans des cellules précurseuses neurales appropriées, un gène promoteur de croissance qui met les cellules neurales en mesure de croître et qui est régi par un facteur extérieur.

10. Lignée cellulaire neurale immortalisée, pouvant être obtenue en incorporant, dans des cellules du système nerveux embryonnaire, un vecteur rétroviral comprenant un gène promoteur de croissance qui est la région sensible à la température de la souche tsA58 du virus SV40 et un gène codant pour la résistance à une drogue, en cultivant lesdites cellules à une température d'environ 33°C dans des conditions adéquates et pendant un temps suffisant pour que la croissance des cellules se produise, et en cultivant ensuite lesdites cellules dans des conditions appropriées et à une température d'environ 39°C.

11. Lignée de cellules oligodendrocytiques du nerf optique, ts U195, déposée à l'American Type Culture Collection sous le numéro de dépôt CRL9729.

FIG. I

pZIP-Neo SV(X)I

FIG. 2A

SV40 Early Region

FIG. 2B